# EUROPÄISCHE PATENTSCHRIFT

(11) **EP 3 295 882 B1**
(45) Veröffentlichungstag und Bekanntmachung des Hinweises auf die Patenterteilung: **05.06.2019**
(21) Anmeldenummer: 16188734.4
(22) Anmeldetag: 14.09.2016
(51) Int. Cl.: A61B 17/86

(54) **KNOCHENSCHRAUBE**
BONE SCREW
VIS À OS

(43) Veröffentlichungstag der Anmeldung: 21.03.2018
(73) Patentinhaber: Grow-Ing Breitenstein, 4410 Liestal (CH)
(72) Erfinder: Breitenstein, Michael, 4493 Wenslingen (CH)
(74) Vertreter: Herrmann, Johanna

(56) Entgegenhaltungen:
- EP-A2- 2 749 236
- US-A- 2 532 296

## Beschreibung

Gegenstand der Erfindung ist eine Knochenschraube, insbesondere eine permanente und/oder degradierbare Leichtbau-Osteosyntheseschraube. Eine derartige Knochenschraube umfasst zwei oder mehrere Knochenschraubenelemente zur Fixation von Knochenfragmenten miteinander oder zueinander. Die Fixation kann mittels Ostesosynthesematerialien erfolgen. Eine derartige Osteosyntheseschraube kann zur Fixation von bleibenden Implantaten im Knochen in ärztlichen, oder zahnärztlichen Anwendungen verwendet werden.

Eine Vielzahl von Knochenschrauben ist auf dem Markt für verschiedenste Anwendungen erhältlich. Knochenschrauben werden zur Verschraubung von frakturierten Knochenfragmenten mit Platten, die dem Zweck der Osteosynthese dienen, oder zur direkten Verschraubung von Knochenfragmenten untereinander ohne Platten verwendet. Anwendung finden derartige Knochenschrauben hauptsächlich in der Traumatologie (Unfallchirurgie) oder in der Umstellungs- und Korrekturosteotomie. Des Weiteren finden Knochenschrauben Anwendung in der Zahnmedizin zum Halten von Zahnersatz im Knochen.

DE10200610060933 A1 zeigt eine typische Knochenschraube, wie auch FR2902637 A1. Diese Schrauben zeigen alle einen zylindrischen Grundkörper mit einem Schraubenschaft, der im Knochen verankert wird und auf dem ein Gewinde aufgebracht ist, um die Schrauben im Knochen formschlüssig zu verankern. DE 43 43 117 A1 zeigt eine Knochenschraube mit einem mit Rillen oder Gewinden ausgestatteten Schraubenkopf, über den die Schraube mittels eines Werkzeuges in den Knochen eingedreht wird. Der Schraubenschaft erstreckt sich vom Schraubenkopf bis zur Schraubenspitze, die je nach Verwendungszweck der Schraube als ein stumpfes oder spitzes Schraubenende sein kann und auch mit einer Schneidnut versehen sein kann (wie die US20140005728 A1 zeigt). In der Ausführungsform als gewindeschneidende Schraube können mehrere Gewindeabschnitte vorgesehen sein (wie die WO2007/048267 A1 zeigt). Der an die Schraubenspitze anschliessende Gewindeabschnitt ist als Vorformbereich ausgebildet, welcher das Gewinde in das Substrat, beispielsweise einen Knochen schneidet. Der Aussendurchmesser des Gewindes im Vorformbereich nimmt mit zunehmendem Abstand von der Schraubenspitze zu. An den Vorformbereich schliesst ein Zwischenbereich an, wobei der Aussendurchmesser des Gewindes im Zwischenbereich wieder abnehmen kann, sodass mit zunehmender Einschraublänge das Drehmoment nicht weiter zunimmt oder weniger stark zunimmt.

Die meisten am Markt angebotenen Schrauben, wie die oben genannten Ausführungsbeispiele, sind aus Vollmaterial hergestellt und werden durch Drehen, Stossen, Umformen oder Fräsen eines Stabrohlings auf eine gewünschte Endform gebracht. Dabei wird oft über 50% des Rohmaterials zerspant, so dass der Materialverbrauch sehr hoch ist.

Die EP1609560 A1 greift diese Problematik auf und offenbart ein Verfahren zur Herstellung eines Einschneidwerkzeugs zum Eindrehen in ein Substrat, welches aus einem Rohrabschnitt und einen Plattenabschnitt aufweist. Der Plattenabschnitt besteht aus zwei länglichen Plattenelementen, an deren Seitenkanten ein Rippenprofil angebracht ist. Die beiden länglichen Plattenelemente weisen je einen Längsschlitz auf, der es ermöglicht die beiden länglichen Plattenelemente zusammenzustecken. Die Plattenelemente können in einem Stanzverfahren hergestellt werden. Die zusammengesteckten Plattenelemente werden in einem Rohrabschnitt aufgenommen. Das Einschneidwerkzeug ist aus einer Vielzahl von Einzelteilen zusammengesetzt. Nachteilig an dem in EP1609560 A1 gezeigten Einschneidwerkzeug ist der aufwändige Produktionsablauf mit einer Vielzahl von Produktionsschritten, welche für viele verschiedene Einzelteile und Verbindungen erforderlich sind. Daher dauert die Herstellung dieses Einschneidwerkzeugs lange und ist teuer.

Die US 2532296 zeigt eine stark vereinfachte Knochenschraube, die aus drei sternförmig zueinander angeordneten Plattenelementen besteht, die an der Aussenseite mit einem Gewinde versehen sind. Es wird in der US 2532296 aber nicht offenbart, wie die Knochenschraube hergestellt wird, sodass aufgrund der gezeigten Geometrie davon auszugehen ist, dass auch diese Knochenschraube durch ein spanabhebendes Verfahren hergestellt worden ist, wobei insbesondere die Herstellung der drei Längskerben nach dem Schneiden des Gewindes durch ein Fräsverfahren erfolgt, wodurch mehr als 50% des Materials verloren gehen.

Somit ist die Schraubenfertigung mit einem hohen Material-, Zeit- und Kostenaufwand verbunden. Daher besteht Bedarf an Knochenschrauben, die mittels kostengünstigeren ressourcenschonenden Herstellmethoden hergestellt werden können.

Eine Aufgabe der Erfindung ist es, eine Knochenschraube zu konzipieren, welche einfach herstellbar und in einem einzigen Arbeitsschritt einfach im Knochen implantierbar ist. Eine weitere Aufgabe der Erfindung ist es, eine Knochenschraube bereitzustellen, welche mit dem Knochen verwachsen kann und dementsprechend im Körper verbleiben kann.

Die Lösung der Aufgaben der Erfindung erfolgt durch die Merkmale des Anspruchs 1. Weitere vorteilhafte Ausführungen sind Gegenstand der Ansprüche 2 bis 10.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für die erfindungsgemässe Knochenschraube. Die Knochenschraube kann mittels unterschiedlicher Herstellungsverfahren hergestellt werden, sodass die Beschreibung eines bestimmten Herstellungsverfahrens nur als beispielhaft anzusehen ist. In der Beschreibung und den Ansprüchen werden die Begriffe "enthalten", "umfassen", "aufweisen" als "enthalten, aber nicht beschränkt auf.." interpretiert.

Eine Knochenschraube enthält ein erstes Knochenschraubenelement, ein zweites Knochenschraubenelement. Die Knochenschraube kann auch mindestens ein weiteres Knochenschraubenelement enthalten. Das erste Knochenschraubenelement und das zweite Knochenschraubenelement sind als dünnwandiges Plattenelement ausgebildet. Das dünnwandige Plattenelement weist eine Wandstärke auf, die weniger als 10% der Länge des Plattenelements beträgt. Jedes der ersten oder zweiten Knochenschraubenelemente besteht aus einem Schraubenkopfelement und einem Schraubenschaftelement. Das Schraubenschaftelement weist eine erste Seitenflanke und eine zweite Seitenflanke auf, wobei jede der Seitenflanken ein Gewindeprofil aufweist.

Nach einem Ausführungsbeispiel kann das dem Schraubenkopfelement gegenüberliegende Ende des Knochenschraubenelements als ein Schraubenspitzenelement ausgebildet sein.

Das erste Knochenschraubenelement und das zweite Knochenschraubenelement können einen ersten und einen zweiten Längsschlitz aufweisen, die sich nicht über die gesamte Länge des Knochenschraubenelements erstrecken. Insbesondere kann der Längsschlitz des ersten Knochenschraubenelements sich vom Schraubenspitzenelement bis zu einem Kreuzungspunkt erstrecken. Der Längsschlitz des zweiten Knochenschraubenelements kann sich vom Schraubenkopfelement bis zu einem Kreuzungspunkt erstrecken. Das erste Knochenschraubenelement kann im zweiten Längsschlitz aufgenommen sein und das zweite Knochenschraubenelement kann im ersten Längsschlitz aufgenommen sein.

Nach einem Ausführungsbeispiel können das erste Knochenschraubenelement und das zweite Knochenschraubenelement als ein spiralförmiges Plattenelement ausgebildet sein.

Nach einem Ausführungsbeispiel sind das erste und zweite Knochenschraubenelement kreuzweise zueinander angeordnet.

Nach einem Ausführungsbeispiel ist ein drittes Knochenschraubenelement vorgesehen, wobei das erste, zweite und dritte Knochenschraubenelement sternförmig zueinander angeordnet sind.

Nach einem Ausführungsbeispiel ist das Gewindeprofil als Sägezahnprofil ausgebildet.

Als Werkstoff für die Knochenschraube wird vorzugsweise ein biokompatibles Material verwendet. Die Knochenschraube kann ein biokompatibles Material enthalten. Insbesondere kann zumindest die Oberfläche der Knochenschraubenelemente aus einem biokompatiblen Material bestehen oder mit einem biokompatiblen Material beschichtet sein. Als biokompatibel bezeichnet man Werkstoffe oder Baugruppen, die keinen negativen Einfluss auf Lebewesen in ihrer Umgebung haben. Biokompatible Materialien können Metalle, Polymere, oder Keramiken sein.

Besonders bevorzugt wird ein metallischer Werkstoff aus der Gruppe von Magnesium, Titan oder aus der Gruppe der Magnesiumlegierungen oder Titanlegierungen verwendet.

Das Metall kann ein Element aus der Gruppe der biokompatibilitätsgeprüften rostfreien Stähle aus Chrom-Nickel und Chrom-Molybdän Legierungen enthalten, beispielsweise ein Element der Gruppe bestehend aus X42CrMo15, X100CrMo17, X2CrNiMnMoNNb21-16-5-3, X20Cr13, X15Cr13, X30Cr13 ,X46Cr13, X17CrNi16-2, X14CrMoS17, X30CrMoN15-1, X65CrMo 17-3, X55CrMo14, X90CrMoV18 ,X50CrMoV15, X 38CrMo V15, G-X 20CrMo13, X39CrMo17-1, X40CrMoVN16-2, X105CrMo17, X20CrNiMoS13-1, X5CrNi18-10, X8CrNiS18-9.

Ein biokompatibilitätsgeprüftes Titan kann ein Element der Gruppe der Reintitane Grade1-4 oder Titanlegierungen Nitinol enthalten. Die Titanlegierung kann ein Element aus der Gruppe der Ti6Al4V oder Ti6Al7Nb enthalten.

Gemäss einem Ausführungsbeispiel kann die Knochenschraube ein Polymer enthalten, insbesondere ein biokompatibilitätsgeprüftes Polymer. Das biokompatibilitätsgeprüfte Polymer kann einen thermoplastischen Kunststoff enthalten. Das biokompatibilitätsgeprüfte Polymer kann ein Element der Gruppe der Acrylat-Copolymerisate, Styrol-Copolymere, Cellulose, Cellulosederivate, Copolyester, Ethylen-Copolymerisate, Fluorpolymere enthalten. Das biokompatibilitätsgeprüfte Polymer kann insbesondere ein Element der Gruppe der MBS, PMMI, MABS, CA, CTA, CAB, CAP, COC, PCT, PCTA, PCTG, EVA, EVAL, PTFE, ePTFE, PCTFE, PVDF, PVF, ETFE, ECTFE, FEP, PEEK, POM, PET enthalten. Das heisst, das biokompatibilitätsgeprüfte Polymer kann ein Element der Gruppe der 3-Maleimidobenzoesäure-N-hydroxysuccinimid-Ester (MBS), Polymethacrylmethylimid (PMMI), Methylmethacrylat-Acrynitril-Butadien-Styrol-Polymerisate (MABS), Celluloseacetate (CA), Cellulosetriacetate (CTA), Celluloseacetobutyrate (CAB), Celluloseacetopropionate (CAP), Cycloolefin-Copolymere (COC), Copolyester (PCT, PCTA, PCTG), Ethylen-VinylacetatCopolymerisate (EVA), Ethylen-Vinylalkohol-Copolymerisate (EVAL), Polytetrafluorethylene (PTFE), expandierte Polytetrafluoroethylene (ePTFE), Polyfluortrichlorethylene (PCTFE), Polyvinylidenfluoride (PVDF), Polyvinylfluoride (PVF), Ethylen-Tetrafluorethylen-Copolymere (ETFE), Ethylen-Chlortrifluorethylen-Copolymere (ECTFE), Polyflour-Ethylen-Propylene oder Polyhexafluorpropylene (FEP), Polyetheretherketone (PEEK), Polyoxymethylene (POM), Polyethylenterephtalate (PET) enthalten.

Gemäss einem Ausführungsbeispiel kann die Knochenschraube eine Keramik enthalten, insbesondere eine biokompatibilitätsgeprüfte Keramik. Die biokompatibilitätsgeprüfte Keramik kann ein Element aus der Gruppe der Al203, Y-TZP (TZP), AMC, HA, TCP, FZM/K, TZP-A, ATZ, C799 enthalten.

Insbesondere kann zumindest ein Element der Gruppe der rostfreien Stähle und Titanlegierungen für nichtauflösende Schrauben zum Einsatz kommen. Insbesondere kann zumindest ein Element der Gruppe der Magnesiumlegierungen oder der Hydroxylapatitverbindungen für resorbierbare Schrauben zum Einsatz kommen.

Die Knochenschraubenelemente können formschlüssig und/oder mittels eines Fügeverfahrens wie z.B. Schweissen oder Löten materialschlüssig verbunden sein. Insbesondere können die Knochenschraubenelemente nach dem Fügen spiralförmig verdreht werden.

Wenn die Knochenschraubenelemente formschlüssig miteinander verbunden sind, greifen die Knochenschraubenelemente ineinander ein und bilden dergestalt Verbindungspartner aus, die ohne Kraftaufwand nicht mehr gelöst werden können. Im eingesetzten Zustand wirken Zugkräfte oder Druckkräfte im Wesentlichen normal zur Längsachse der Knochenschraube, sodass in Richtung der Längsachse der Knochenschraube keine Kräfte oder nur sehr geringe Kräfte wirken. Die materialschlüssige Verbindung ist eine feste nicht lösbare Verbindungstechnik, die bei Metallen vor allem über Laserschweissen erfolgt, da hierbei kein Material zugefügt werden muss, sondern das Rohmaterial der Verbindungspartner punktuell aufgeschmolzen wird und eine tragfähige Verbindung geschaffen wird.

Die Knochenschraube nach einem der Ausführungsbeispiele kann über ein vorgebohrtes Loch im Knochen eingebracht werden. Dies erfolgt entweder durch konventionelles Einschrauben mittels eines Einschraubwerkzeugs oder durch Einschlagen und einer nachfolgenden Fixation mit einer definierten Drehbewegung. Wenn die Knochenschraube ohne Vorschneiden eines Gewindes in den Knochen eingebracht wird, handelt es sich um eine selbstschneidende Knochenschraube.

Das Einbringen der Knochenschraube kann auch ohne Vorbohren erfolgen. In diesem Fall bildet das Schraubenspitzenelement den Bohrer aus, mittels welchem das Bohrloch im Knochen erzeugt wird. Gleichzeitig mit dem Bohren des Bohrlochs wird die Knochenschraube in den Knochen eingebracht. In diesem Fall wird die Knochenschraube als selbstbohrende Knochenschraube eingesetzt.

Die Oberfläche der Knochenschraubenelemente kann glatt und poliert sein, oder eine definierte Rauigkeit aufweisen, was z.B. mit einer abrasiven Oberflächenbehandlung erreicht wird. Eine definierte Rauigkeit hat den Vorteil, dass sich auf der Oberfläche des Knochenschraubenelements Knochenzellen besonders gut ansiedeln können. Im diesem Fall spricht man von Osteoinduktion, das heisst einer Knochenneubildung durch Stimulierung der Differenzierung knochenbildender Zellen im Lagergewebe. Es gibt noch die Möglichkeit, die Oberfläche der Knochenschraube mit wachstumsfördernden Stoffen wie Proteinen zu beschichten, was ebenfalls zu Osteoinduktion führt. Die Knochenschraube kann auch mit Medikamenten beschichtet werden, wie z.B. Entzündungshemmern, um ein problemloses Einwachsen zu gewährleisten.

Insbesondere bedingt durch die kreuzförmige resp. sternförmige Anordnung der Knochenschraubenelemente kann der Knochen sehr gut an die Knochenschraube heranwachsen und ermöglicht eine stabile Fixation insbesondere bei Implantaten, die im Körper verbleiben. Dies ist im Vergleich zu den bestehenden Knochenschrauben vorteilhaft, da die mit Knochen bewachsene Oberfläche grösser als bei einer konventionellen zylindrischen Knochenschraube ist.

Das Schraubenspitzenelement kann je nach Knochen und Anwendung rund, flach, konisch oder spitz ausgeführt sein und/oder kann mit einer Zentrierspitze und/oder Anbohrspitze versehen sein.

Das Gewinde ist vorzugsweise derart geformt, dass es eine gute Verankerung im Knochen, besonders im kortikalen Teil gewährleistet. Zudem kann das Gewinde für die Wirkung von Zugkräften aufgrund eines asymmetrischen Profils optimiert sein. Das Gewinde weist somit auf jedem der Knochenschraubenelemente ein Gewindeprofil auf. Das Gewindeprofil enthält eine vordere Gewindeflanke und eine hintere Gewindeflanke. Die vordere Gewindeflanke kann einen spitzeren Winkel als die hintere Gewindeflanke aufweisen. Unter dem Winkel ist der Winkel zu verstehen, den die Gewindeflankenebene mit der Längsachse einschliesst. Unter der vorderen Gewindeflanke ist die Gewindeflanke zu verstehen, die in Richtung des Schraubenspitzenelements ausgerichtet ist. Unter der hinteren Gewindeflanke ist die Gewindeflanke zu verstehen, die in Richtung des Schraubenkopfelements ausgerichtet ist, betrachtet vom Schraubenspitzenelement in Richtung Schraubenkopfelement. Insbesondere kann ein Sägezahnprofil ausgebildet werden. Der Winkel wird zwischen der Flanke und einer gedachten Ebene gemessen, die senkrecht auf dem Durchmesser steht. Dabei entspricht der Durchmesser im Wesentlichen der Breite des Knochenschraubenelements.

Das Gewinde kann eine gleichmässige oder eine ungleichmässige Steigung aufweisen - je nachdem, ob die Knochenschraube für die Kompression von Platten auf Knochen oder zur Verbindung von einem Knochen mit einem weiteren Knochen verwendet wird. Eine vorbekannte Ausführung ist die Herbert-Schraube, die in abgewandelter Form durch Verwendung eines ersten und zweiten Knochenschraubenelements durch die Erfindung erhältlich ist. Die typische Herbert-Schraube wird als Knochenkompressionsschraube ohne Platte verwendet. Entsprechend weist jedes der Knochenschraubenelemente einen Schraubenschaft und kurze Gewindeabschnitte am Anfang und am Ende des Schraubenschafts auf. Die Herbert-Schraube wird in der Literatur auch als Doppelgewindeschraube bezeichnet. Der Durchmesser der beiden Gewindeabschnitte bei einer zylinderförmigen Herbert-Schraube ist etwas grösser ist als der des Schraubenschaftes. Entsprechend ist die Breite der beiden Gewindeabschnitte der Knochenschraubenelemente der erfindungsgemässen Knochenschraube grösser als die Breite des Schraubenschafts. Ein Schraubenkopf ist nicht erforderlich, sodass die Knochenschraube einfacher vollständig im Knochen platziert werden kann. Damit eine axiale Zugkraft auf die Gewindeabschnitte entsteht, ist die Gewindesteigung am proximalen Gewindeabschnitte kleiner als am distalen, sodass der distale Gewindeabschnitt mechanisch an den proximalen Gewindeabschnitt beim Einschrauben herangezogen werden kann. Eine derartige Knochenschraube wird daher als Zugschraube verwendet. Dadurch entsteht ein die Bruchheilung begünstigender Druck auf den Bruchspalt, was in der Literatur auch unter dem Begriff interfragmentäre Kompression bekannt ist.

Ebenfalls kann nach einem Ausführungsbeispiel der Gewindeausssendurchmesser und/oder der Gewindekerndurchmesser konstant sein. Nach einem Ausführungsbeispiel kann der Gewindeausssendurchmesser und/oder der Gewindekerndurchmesser am Schraubenkopfelement oder am Schraubenschaftelement variieren. Hierdurch können die Gewinde miteinander verkeilt werden. Diese Anwendung ist bei winkelstabilen variablen Verschraubungen für eine Knochenplatte zur Ostheosynthese und dem zugehörigen Implantat vorteilhaft.

Die Dimensionen der Knochenschraube können von einem Aussendurchmesser von 2 mm bei einer Schraube für Anwendungen im Mittelgesicht, bis zu einem Aussendurchmesser von 7 mm für Anwendungen im Bereich der Wirbelsäule gehen. Die Länge der Knochenschraube kann 4 mm bis zu 80 mm betragen.

Nach einem Ausführungsbeispiel weist das Schraubenkopfelement eine maximale Breite auf, die grösser ist als die maximale Breite des Gewindeprofils ist. Insbesondere kann die maximale Breite des Schraubenkopfelements mindestens 10% grösser sein als die maximale Breite des Gewindeprofils.

Der Materialverbrauch für die Herstellung der erfindungsgemässen Knochenschraube ist im Vergleich zum Stand der Technik mindestens um den Faktor 4 verringert. Für die Herstellung einer Knochenschraube gemäss US 2532296 wird mehr als 50% des Rohlings abgetragen und fällt als recyclierbarer Abfall an. Für eine Knochenschraube gemäss der vorliegenden Erfindung kann das Rohmaterial in Enddicke als Ausgangsmaterial für das dünnwandige Plattenelement verwendet werden und durch Laserschneiden, Wasserstrahlschneiden, oder durch Stanzen hergestellt werden. Dabei fällt sehr wenig Abfall an, da die Schraubenquerschnitte ineinander geschachtelt werden können.

Ein weiterer Nachteil der konventionellen Schraubenfertigung ist der grosse Zeitaufwand, der durch das Einrichten der Drehmaschine, den Fertigungsprozess des Drehens an sich, sowie den mehrstufigen Reinigungsprozess benötigt wird, der insbesondere durch die Verwendung eines Kühlmediums beim Drehen erforderlich ist.

Gemäss der vorliegenden Erfindung kann jedes der Knochenschraubenelemente in einem einzigen Arbeitsschritt hergestellt werden. In einem zweiten Arbeitsschritt werden die Knochenschraubenelemente zu einer Knochenschraube zusammengebaut. Der Zusammenbau der Knochenschraubenelemente kann durch eine formschlüssige Verbindung erfolgen, wie beispielsweise eine Steckverbindung, oder eine kraftschlüssige Verbindung umfassen, die beispielsweise durch Laserschweissen hergestellt ist.

Ein weiterer Nachteil einer herkömmlichen Knochenschraube, die als zylindrischer oder konischer Vollkörper mit umlaufendem Gewinde ausgebildet ist, ist die Menge des körperfremden Materials, das dem Patienten implantiert wird. Auch wenn bisher keine allergische Reaktion von Titan im Körper nachgewiesen wurde, befindet sich im Körper des Patienten Metall, auf das der Patient mit unterschiedlicher Sensibilität reagieren kann. Dieser Effekt kann durch die vorliegende Erfindung stark reduziert werden, weil der Knochen wesentlich weniger Metall enthält.

Ein weiterer Nachteil der konventionellen Schraube ist die Explantation, die in den meisten Fällen vorgenommen werden muss. Der Patient muss sich einer zweiten Operation unterziehen und je nach dem, wie stark die Schrauben eingewachsen sind, ist es aufwändig, sie gegebenenfalls invasiv wieder zu entfernen. Die vorliegende Erfindung bietet den Knochenzellen eine grosse Oberfläche zum Anhaften und wächst daher sehr schnell ein und ermöglicht bedingt durch den Querschnitt eine formschlüssige Verbindung mit dem Knochensubstrat zu erzielen. Zudem hat die Schraube in einer Ausführung aus resorbierbarem Material auch den Vorteil, dass die Schrauben nicht wieder entfernt werden müssen und daher keine erneute Operation nötig ist. Die konventionelle Schraube hat auch den Nachteil, dass die Verbindung zum Einbringwerkzeug über eine formschlüssige Ausnehmung, beispielsweise eine Nut im Kopf der Schraube erfolgt. Diese Ausnehmung muss kleiner als der Kopf der Schraube sein und ermöglicht beim Setzen der Schraube daher nur eine begrenzte Kraftübertragung. Eine Knochenschraube gemäss der vorliegenden Erfindung kann einen Schraubenkopf mit grosser Querschnittsfläche aufweisen, sodass auch grosse Kräfte übertragen werden können, gegebenenfalls ohne Einsatz eines Hilfswerkzeugs.

Eine Knochenschraube gemäss der vorliegenden Erfindung hat gegenüber den bekannten Lösungen vor allem die nachfolgend angeführten Vorteile.

Die Herstellung der Knochenschraube ist sehr einfach und kostengünstig.

Die Knochenschraube ist im Vergleich zu herkömmlichen Knochenschrauben ein ressourcenschonendes Produkt, weil im Vergleich zu herkömmlichen Knochenschrauben weniger als 50% Material benötigt wird.

Die Verankerung im Knochen ist insbesondere durch die kreuzförmige oder sternförmige Querschnittsform gegenüber einer zylinderförmigen oder konischen Knochenschraube wesentlich verbessert.

Die Knochenschraube bietet einen idealen Formschluss für das Einschraubwerkzeug und kann gegenüber den bekannten Lösungen kaum vom Einschraubwerkzeug abfallen, weil die Haltefläche der Einschraubwerkzeughalterung mit der umgekehrten Geometrie der bekannten Kreuzschlitzverbindung sehr gross ist und das Einschraubwerkzeug je nach gefertigter Genauigkeit mit der Schraubenfläche einen Reibschluss ausbildet. Zusätzlich können die Innenflächen der Einschraubwerkzeughalterung leicht konisch ausgebildet sein, um den Reibschluss durch Klemmwirkung noch zu verbessern.

Beim Einschrauben der Knochenschraube können grosse Kräfte übertragen werden, weil sie im Vergleich mit der bekannten Kreuzschlitzverbindung, dessen Umkehrform sie bildet, eine um ein Mehrfaches grössere Übertragungsfläche zwischen Werkzeug und Schraube aufweist.

Nachfolgend wird die erfindungsgemässe Knochenschraube anhand einiger Ausführungsbeispiele dargestellt. Es zeigen
Fig. 1 eine Ansicht der Knochenschraubenelemente einer Knochenschraube nach einem ersten Ausführungsbeispiel,
Fig. 2 eine Ansicht einer Knochenschraube nach dem ersten Ausführungsbeispiel,
Fig. 3 eine Seitenansicht der Knochenschraube nach dem ersten Ausführungsbeispiel,
Fig. 4 eine Ansicht einer Knochenschraube nach einem zweiten Ausführungsbeispiel,
Fig. 5 eine Ansicht einer Knochenschraube nach einem dritten Ausführungsbeispiel,
Fig. 6 eine Draufsicht auf die Knochenschraube gemäss Fig. 5,
Fig. 7 eine Ansicht der Knochenschraube gemäss des dritten Ausführungsbeispiels im entkoppelten Zustand,
Fig. 8 eine Ansicht der Knochenschraube des dritten Ausführungsbeispiels im entkoppelten Zustand in einer Seitenansicht,
Fig. 9 eine Ansicht der Knochenschraube des dritten Ausführungsbeispiels im entkoppelten Zustand in einer weiteren Seitenansicht in Einbaulage,
Fig. 10 eine Ansicht einer Knochenschraube nach einem vierten Ausführungsbeispiel,
Fig. 11 eine Seitenansicht der Knochenschraube gemäss Fig. 10,
Fig. 12 eine Seitenansicht der Knochenschraube gemäss Fig. 10 mit Einschraubwerkzeug.

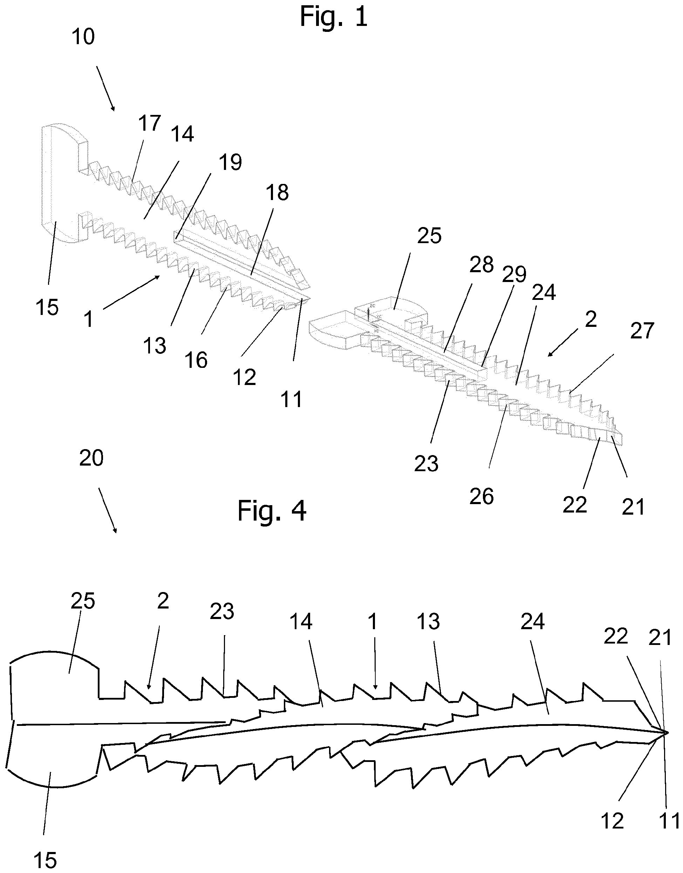

Fig. 1 zeigt eine erste Ansicht einer Knochenschraube 10, enthaltend ein erstes Knochenschraubenelement 1, ein zweites Knochenschraubenelement 2, wobei das erste Knochenschraubenelement 1 und das zweite Knochenschraubenelement 2 als dünnwandiges Plattenelement ausgebildet sind. Das dünnwandige Plattenelement weist eine Wandstärke auf, die weniger als 10% der Länge des Plattenelements beträgt. Das ersten Knochenschraubenelement 1 besteht aus einem Schraubenkopfelement 15 und einem Schraubenschaftelement 14. Das zweite Knochenschraubenelement 2 besteht aus einem Schraubenkopfelement 25 und einem Schraubenschaftelement 24. Jedes der ersten oder zweiten Schraubenschaftelemente 14, 24 weist eine erste Seitenflanke 16, 26 und eine zweite Seitenflanke 17, 27 auf, wobei jede der Seitenflanken ein Gewindeprofil aufweist.

Das dem Schraubenkopfelement 15 gegenüberliegende Ende des Knochenschraubenelements 1 ist als ein Schraubenspitzenelement 12 ausgebildet. Das dem Schraubenkopfelement 25 gegenüberliegende Ende des Knochenschraubenelements 2 ist als ein Schraubenspitzenelement 22 ausgebildet.

Das erste Knochenschraubenelement 1 weist einen ersten Längsschlitz 18 auf, der sich nicht über die gesamte Länge des Knochenschraubenelements erstreckt. Das zweite Knochenschraubenelement 2 weist einen zweiten Längsschlitz 28 auf, der sich nicht über die gesamte Länge des Knochenschraubenelements erstreckt.

Der Längsschlitz 18 des ersten Knochenschraubenelements 1 erstreckt sich vom Schraubenspitzenelement 12 bis zu einem Kreuzungspunkt 19. Der Längsschlitz des zweiten Knochenschraubenelements 2 erstreckt sich vom Schraubenkopfelement 25 bis zu einem Kreuzungspunkt 29.

Das Schraubenkopfelement 15, 25 kann eine maximale Breite aufweisen, die grösser ist als die maximale Breite des Gewindeprofils. Das Schraubenkopfelement 15, 25 kann somit das entsprechende Schraubenschaftelement 14, 24 überragen. Das heisst der Normalabstand zwischen zwei auf einem der Knochenschraubenelemente 1, 2 jeweils gegenüberliegend angeordneten von je zwei benachbarten Gewindeflanken gebildeten Gewindespitzen ist kleiner als die Breite des entsprechenden Schraubenkopfelements 15, 25. Die Breite des Schraubenkopfelements 15, 25 wird in Normalrichtung zur Längsachse gemessen. Die Länge des Schraubenkopfelements, das heisst, dessen Abmessung in Richtung der Längsachse der Knochenschraube, kann kleiner, gleich gross oder grösser sein als die maximale Breite des Schraubenkopfelements 15, 25. Nach einem Ausführungsbeispiel ist die Länge des Schraubenkopfelements 15, 25 kleiner als die Breite des Schraubenkopfelements 15, 25.

Jedes der Schraubenkopfelemente 15, 25 enthält somit je zwei gegenüberliegende Seitenflächen. Diese Seitenflächen können zum Montage in einer entsprechenden kreuzförmigen Nut eines Montagewerkzeugs aufgenommen werden. Diese Nut des Montagewerkzeugs kann sich zumindest über einen Teil der Länge des Schraubenkopfelements erstrecken. Wenn die Nut des Montagewerkzeugs geeignet ist, die gesamte Länge des Schraubenkopfelements aufzunehmen, werden pro Schraubenkopfelement je zwei Kontaktflächen ausgebildet, die dem Montagewerkzeug guten Halt bieten. Hierdurch kann das Setzen oder die Entnahme der Knochenschraube vereinfacht werden, da die durch die Seitenflächen der Schraubenkopfelemente 15, 25 gebildete Werkzeugaufnahme die Übertragung grösserer Kräfte zulässt als eine herkömmliche Verbindung, die mittels eines Schraubenschlüssels, beispielsweise in Form eines Sechskants oder in Torx-Ausführung oder über einen Schlitz oder Kreuzschlitz für einen herkömmlichen Schraubenzieher gesetzt bzw. gelöst wird.

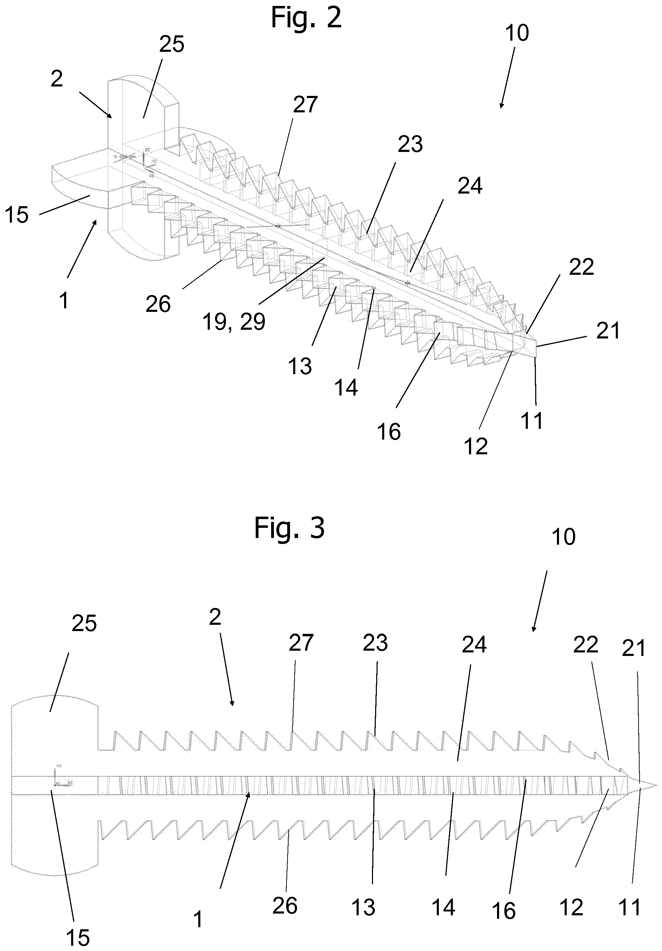

Fig. 2 zeigt eine Ansicht der Knochenschraube 10 im zusammengebauten Zustand. Das erste Knochenschraubenelement 1 ist im zweiten Längsschlitz 28 aufgenommen und das zweite Knochenschraubenelement 2 ist im ersten Längsschlitz 18 aufgenommen. Das erste und zweite Knochenschraubenelement 1, 2 sind kreuzweise zueinander angeordnet.

Fig. 3 zeigt eine Seitenansicht der Knochenschraube 10 im zusammengebauten Zustand. In Fig. 3 ist das zweite Knochenschraubenelement 2 mit seinen beiden Seitenflanken 26, 27 sichtbar. Jede der Seitenflanken 26, 27 enthält ein Gewindeprofil. Das Gewindeprofil ist gemäss diesem Ausführungsbeispiel als sägezahnartiges Gewindeprofil ausgebildet. Die Zacken des sägezahnartigen Gewindeprofils können zwei Kanten aufweisen, die unterschiedliche Steigung aufweisen. Insbesondere weisen die gegen das Schraubenkopfelement 25 ausgerichteten Kanten eine grössere Steigung auf als die gegen das Schraubenspitzenelement 22 ausgerichteten Kanten. Alternativ kann die Steigung konstant bleiben, sich aber der Durchmesser ändern. Insbesondere kann der Durchmesser spiralförmig grösser werden. Mit anderen Worten nimmt der Durchmesser der Spirale, welche durch die durch die Spitzen der vorderen und hinteren Gewindeflanken ausgebildet wird, vom Schraubenspitzenelement 22 in Richtung des Schraubenkopfelements 25 zu. Die Spirale ist um die gemeinsame Längsachse der Knochenschraubenelemente 1, 2 gewunden. Die Längsachse wird als Schnittlinie der Mittenebenen der Knochenschraubenelemente 1, 2 erhalten.

Vom ersten Knochenschraubenelement 1 ist nur die erste Seitenflanke 16 sichtbar, die Seitenflanke 17 liegt der ersten Seitenflanke 16 gegenüber und ist daher nur in Fig. 1 sichtbar. Die Kantenflächen der Kanten verlaufen nicht in vertikaler Richtung zur Längsachse, sondern weisen einen Neigungswinkel zu der Vertikalrichtung auf, welche der Steigung des Gewindeprofils entspricht.

Fig. 4 zeigt Ansicht einer Knochenschraube 20, enthaltend ein erstes Knochenschraubenelement 1 und ein zweites Knochenschraubenelement 2, wobei das erste Knochenschraubenelement 1 und das zweite Knochenschraubenelement 2 als dünnwandiges Plattenelement ausgebildet sind. Das dünnwandige Plattenelement weist eine Wandstärke auf, die weniger als 10% der Länge des Plattenelements beträgt. Jedes der ersten oder zweiten Knochenschraubenelemente 1, 2 besteht aus einem Schraubenkopfelement 15, 25 und einem Schraubenschaftelement 14, 24. Das Schraubenschaftelement 14, 24 weist jeweils eine erste Seitenflanke 16, 26 und eine zweite Seitenflanke 17, 27 auf, wobei jede der Seitenflanken ein Gewindeprofil aufweist. Gemäss Fig. 4 sind das erste Knochenschraubenelement 1 und das zweite Knochenschraubenelement 2 durch ein spiralförmiges Plattenelement ausgebildet.

Das dem Schraubenkopfelement 15, 25 gegenüberliegende Ende des Knochenschraubenelements 1, 2 ist als ein Schraubenspitzenelement ausgebildet 12, 22. Das erste Knochenschraubenelement und das zweite Knochenschraubenelement 1, 2 können einen ersten und einen zweiten Längsschlitz 18, 28 aufweisen, die sich nicht über die gesamte Länge des Knochenschraubenelements erstrecken, die aber in der vorliegenden Darstellung nicht sichtbar sind. Der Längsschlitz 18 des ersten Knochenschraubenelements 1 erstreckt sich vom Schraubenspitzenelement 12 bis zu einem Kreuzungspunkt 19. Der Längsschlitz des zweiten Knochenschraubenelements 2 erstreckt sich vom Schraubenkopfelement 25 bis zu einem Kreuzungspunkt 29. Das erste Knochenschraubenelement 1 ist im zweiten Längsschlitz 28 aufgenommen und das zweite Knochenschraubenelement 2 ist im ersten Längsschlitz 18 aufgenommen. Das erste Knochenschraubenelement 1 und das zweite Knochenschraubenelement 2 können zusätzlich zueinander durch eine kraftschlüssige Verbindung, beispielsweise eine Schweissverbindung, miteinander verbunden sein. Das erste Knochenschraubenelement 1 und das zweite Knochenschraubenelement werden um die Längsachse verwunden, sodass sich eine Spiralform ergibt.

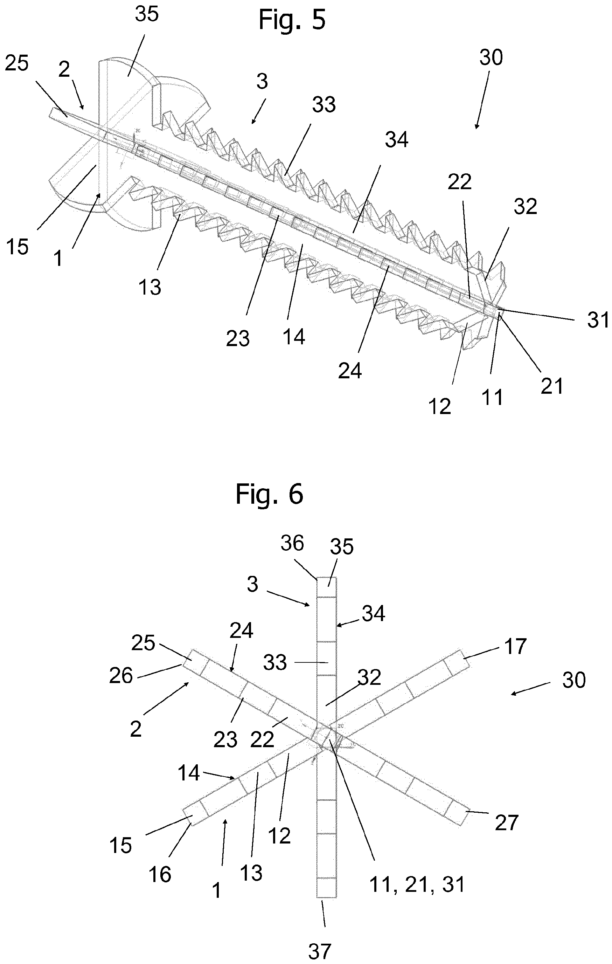

Fig. 5 zeigt eine erste Ansicht einer Knochenschraube 30, enthaltend ein erstes Knochenschraubenelement 1, ein zweites Knochenschraubenelement 2 und ein drittes Knochenschraubenelement 3, wobei das erste Knochenschraubenelement 1, das zweite Knochenschraubenelement 2 und das dritte Knochenschraubenelement 3 als dünnwandiges Plattenelement ausgebildet sind. Das dünnwandige Plattenelement weist eine Wandstärke auf, die weniger als 10% der Länge des Plattenelements beträgt. Jedes der ersten, zweiten oder dritten Knochenschraubenelemente 1, 2, 3 besteht aus einem Schraubenkopfelement 15, 25, 35 und einem Schraubenschaftelement 14, 24, 34. Jedes der Schraubenschaftelemente 14, 24, 34 weist je eine erste Seitenflanke 16, 26, 36 und eine zweite Seitenflanke 17, 27, 37 auf, wobei jede der Seitenflanken ein Gewindeprofil aufweist.

Das dem Schraubenkopfelement 15, 25, 35 gegenüberliegende Ende des Knochenschraubenelements 1, 2, 3 ist als ein Schraubenspitzenelement 12, 22, 32 ausgebildet. Das erste Knochenschraubenelement, das zweite und das dritte Knochenschraubenelement 1, 2, 3 weisen einen ersten und einen zweiten Längsschlitz 18, 28, 38 auf, die sich nicht über die gesamte Länge des Knochenschraubenelements erstrecken. Die Längsschlitze sind in der vorliegenden Darstellung nicht sichtbar. Der Längsschlitz 18 des ersten Knochenschraubenelements 1 erstreckt sich vom Schraubenspitzenelement 12 bis zu einem Kreuzungspunkt 19. Der Längsschlitz 28 des zweiten Knochenschraubenelements 2 erstreckt sich vom Schraubenkopfelement 25 bis zu einem Kreuzungspunkt 29. Der Längsschlitz 38 des dritten Knochenschraubenelements 3 erstreckt sich vom Schraubenkopfelement 35 bis zu einem Kreuzungspunkt 39. Das erste Knochenschraubenelement 1 ist im zweiten Längsschlitz 28 oder im dritten Längsschlitz 38 aufgenommen und das zweite Knochenschraubenelement 2 ist im ersten Längsschlitz 18 oder im dritten Längsschlitz 38 aufgenommen. Das dritte Knochenschraubenelement kann in einem der ersten Längsschlitze 18 oder zweiten Längsschlitze 28 aufgenommen sein.

Fig. 6 zeigt eine Draufsicht auf die Knochenschraube 30 gemäss Fig. 5. Fig. 6 zeigt, dass das erste, zweite und dritte Knochenschraubenelement 1, 2, 3 sternförmig zueinander angeordnet sind.

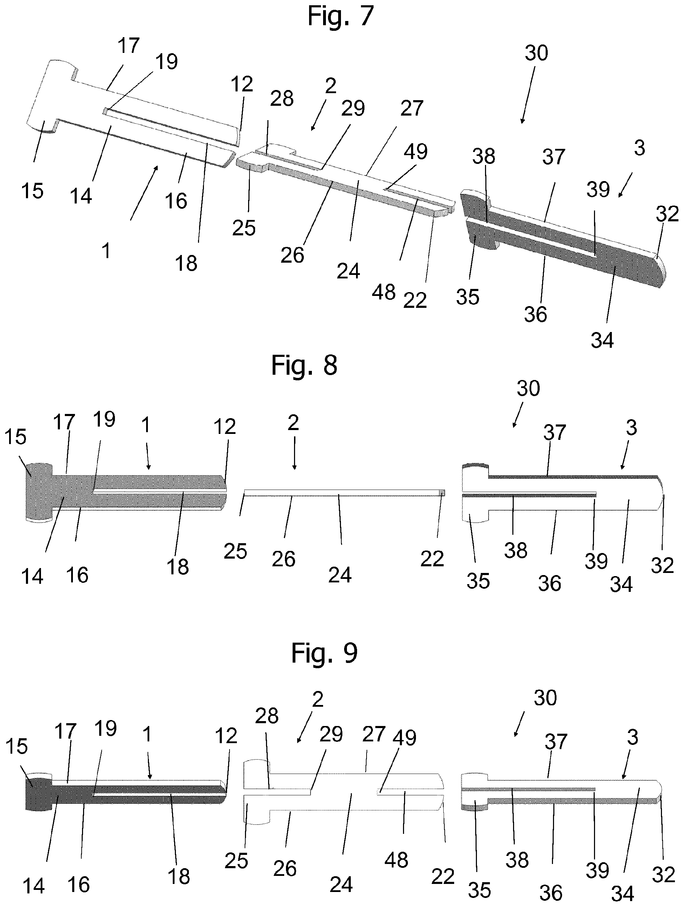

Fig. 7 zeigt eine Ansicht der Knochenschraube 30 gemäss dem dritten Ausführungsbeispiel im entkoppelten Zustand. Das erste Knochenschraubenelement 1, das zweite Knochenschraubenelement 2 und das dritte Knochenschraubenelement 3 sind voneinander getrennt dargestellt. Das erste Knochenschraubenelement 1, das zweite Knochenschraubenelement 2 und das dritte Knochenschraubenelement 3 sind als dünnwandiges Plattenelement ausgebildet. Das dünnwandige Plattenelement weist eine Wandstärke auf, die weniger als 10% der Länge des Plattenelements beträgt. Jedes der ersten, zweiten oder dritten Knochenschraubenelemente 1, 2, 3 besteht aus einem Schraubenkopfelement 15, 25, 35 und einem Schraubenschaftelement 14, 24, 34. Jedes der Schraubenschaftelemente 14, 24, 34 weist je eine erste Seitenflanke 16, 26, 36 und eine zweite Seitenflanke 17, 27, 37 auf, wobei jede der Seitenflanken ein Gewindeprofil aufweist, welches in der vorliegenden Darstellung weggelassen ist.

Das dem Schraubenkopfelement 15, 25, 35 gegenüberliegende Ende des Knochenschraubenelements 1, 2, 3 ist als ein Schraubenspitzenelement 12, 22, 32 ausgebildet. Das erste Knochenschraubenelement, das zweite und das dritte Knochenschraubenelement 1, 2, 3 weisen zumindest einen Längsschlitz 18, 28, 38 auf, die sich nicht über die gesamte Länge des Knochenschraubenelements erstrecken. Der Längsschlitz 18 des ersten Knochenschraubenelements 1 erstreckt sich vom Schraubenspitzenelement 12 bis zu einem Kreuzungspunkt 19. Das zweite Knochenschraubenelement 2 weist zwei Längsschlitze 28, 48 auf. Der erste Längsschlitz 28 erstreckt sich vom Schraubenkopfelement 25 bis zu einem Kreuzungspunkt 29. Der zweite Längsschlitz 48 erstreckt sich vom Schraubenspitzenelement 22 bis zu einem Kreuzungspunkt 49. Der Längsschlitz 38 des dritten Knochenschraubenelements 3 erstreckt sich vom Schraubenkopfelement 35 bis zu einem Kreuzungspunkt 39. Das erste Knochenschraubenelement 1 ist im zusammengebauten Zustand im ersten Längsschlitz 28 des zweiten Knochenschraubenelements 2 aufgenommen. Das dritte Knochenschraubenelement 3 ist im zusammengebauten Zustand im zweiten Längsschlitz 48 des zweiten Knochenschraubenelements 2 aufgenommen. Vorzugsweise erstreckt sich der Längsschlitz 18 des ersten Knochenschraubenelements 1 über ungefähr 2/3 der Länge der Knochenschraube. Vorzugsweise erstreckt sich der Längsschlitz 38 des dritten Knochenschraubenelements 3 über ungefähr 2/3 der Länge der Knochenschraube 30. Der erste Längsschlitz 28 sowie der zweite Längsschlitz 48 des zweiten Knochenschraubenelements erstrecken sich vorzugsweise über ungefähr 1/3 der Länge der Knochenschraube 30. Das zweite Knochenschraubenelement 2 enthält somit zwei Kreuzungspunkte 29, 49, während das erste und dritte Knochenschraubenelement 1, 3 jeweils nur einen Kreuzungspunkt 19, 39 aufweisen.

Fig. 8 zeigt eine Ansicht der Knochenschraube des dritten Ausführungsbeispiels im entkoppelten Zustand in einer Seitenansicht. Fig. 9 zeigt eine Ansicht der Knochenschraube des dritten Ausführungsbeispiels im entkoppelten Zustand in einer weiteren Seitenansicht in Einbaulage.

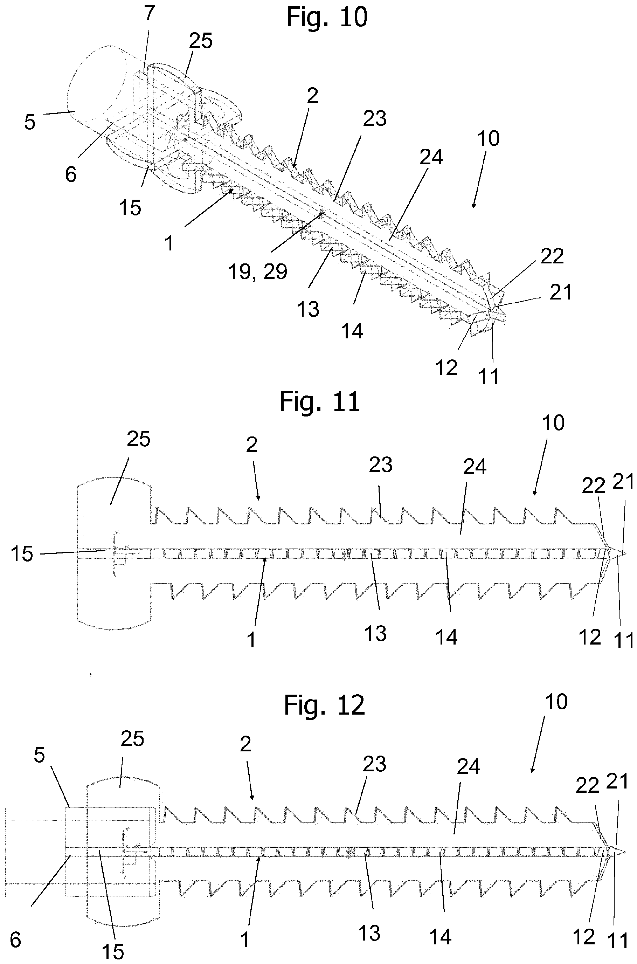

Fig. 10 zeigt eine erste Ansicht der Knochenschraube 10. Die Bauelemente, welche der Darstellung der Fig. 1 entsprechen, tragen wiederum die gleichen Bezugszeichen. Fig. 10 zeigt ein Einschraubwerkzeug 5, welches auf die Schraubenkopfelemente 15, 25 aufgesetzt ist. Das Einschraubwerkzeug 5 kann als zylinderförmiges Element ausgebildet sein, welches zwei kreuzweise zueinander angeordnete Längsschlitze 6, 7 aufweist. Das zylinderförmige Element kann als Vollzylinder oder als Hohlzylinder ausgebildet sein. Wenn der Querschnitt des Einschraubwerkzeugs, gesehen in Normalrichtung zur Längsachse, nicht als ein zylinderförmiges Element ausgebildet ist, kann der Querschnitt auch polygonal, insbesondere sechseckig oder rechteckig ausgeführt sein. Insbesondere kann der Querschnitt als Vierkant oder Sechskant ausgebildet sein, für welchen ein herkömmlicher Schraubenschlüssel verwendet werden kann.

Die Breite der Längsschlitze 6, 7 entspricht im Wesentlichen der Breite des entsprechenden Schraubenkopfelements 15, 25.

Für die Ausführungsform gemäss Fig. 5, 6 kann ein Einschraubwerkzeug verwendet werden, welches drei Längsschlitze aufweist, die sternförmig zueinander angeordnet sind.

Fig. 11 und Fig. 12 zeigen eine Seitenansicht der Knochenschraube 10 im zusammengebauten Zustand. In Fig. 12 ist das Einschraubwerkzeug gezeigt, in Fig. 11 ist die Knochenschraube 10 ohne Einschraubwerkzeug gezeigt. Fig. 11 unterscheidet sich von der Fig. 3 nur dahingehend, dass die Seitenflanken der 12, 22 des Schraubenspitzenelements einen grösseren Neigungswinkel mit der Längsachse einschliessen als in der Ausführung gemäss Fig. 3. Die Anbohrspitze 11, 21 ist in den Varianten gemäss Fig. 3 und Fig. 11 gleich ausgestaltet.

Nach jedem der Ausführungsbeispiele kann das Gewindeprofil als Sägezahnprofil ausgebildet sein. Das Gewindeprofil kann gebohrt, geschnitten, gestanzt oder gefräst werden.

Nach jedem der vorhergehenden Ausführungsbeispiele kann die Knochenschraube mittels eines additiven Herstellungsverfahrens hergestellt werden.

Nach einem nicht dargestellten Ausführungsbeispiel kann das Gewindeprofil abschnittweise vorgesehen sein. Nach jedem der Ausführungsbeispiele kann die Breite des Knochenschraubenelements, welche mit einem Gewindeprofil versehen ist, im Wesentlichen konstant sein. Die Breite des Knochenschraubenelements kann sich auch verändern, insbesondere kann die Breite des Knochenschraubenelements vom Schraubenelement zum Schraubenkopfelement zumindest abschnittsweise zunehmen.

Für den Fachmann ist offensichtlich, dass viele weitere Modifikationen zusätzlich zu den beschriebenen Ausführungsbeispielen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C... N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Knochenschraube (10, 20, 30,), enthaltend ein erstes Knochenschraubenelement (1), ein zweites Knochenschraubenelement (2, 3), wobei das erste Knochenschraubenelement (1) und das zweite Knochenschraubenelement (2, 3) als dünnwandiges Plattenelement ausgebildet sind, wobei das dünnwandige Plattenelement eine Wandstärke aufweist, die weniger als 10% der Länge des Plattenelements beträgt, wobei jedes der ersten oder zweiten Knochenschraubenelemente (1, 2, 3) ein Schraubenkopfelement (15, 25, 35) und ein Schraubenschaftelement (14, 24, 34) enthält, **dadurch gekennzeichnet, dass** das Schraubenschaftelement (14, 24, 34) eine erste Seitenflanke (16, 26, 36) und eine zweite Seitenflanke (17, 27, 37) aufweist, wobei jede der Seitenflanken ein Gewindeprofil aufweist.

2. Knochenschraube nach Anspruch 1, wobei das dem Schraubenkopfelement (15, 25, 35) gegenüberliegende Ende des Knochenschraubenelements (1, 2, 3) als ein Schraubenspitzenelement ausgebildet (12, 22, 32) ist.

3. Knochenschraube nach einem der Ansprüche 1 oder 2, wobei das erste Knochenschraubenelement und das zweite Knochenschraubenelement (1, 2, 3) einen ersten und einen zweiten Längsschlitz (18, 28, 38, 48) aufweisen, die sich nicht über die gesamte Länge des Knochenschraubenelements erstrecken.

4. Knochenschraube nach Anspruch 3, wobei der Längsschlitz (18) des ersten Knochenschraubenelements (1) sich vom Schraubenspitzenelement (12) bis zu einem Kreuzungspunkt (19) erstreckt.

5. Knochenschraube nach einem der Ansprüche 3 oder 4, wobei der Längsschlitz des zweiten Knochenschraubenelements (2) sich vom Schraubenkopfelement (25) bis zu einem Kreuzungspunkt (29) erstreckt.

6. Knochenschraube nach Anspruch 5, wobei das erste Knochenschraubenelement (1) im zweiten Längsschlitz (28) aufgenommen ist und das zweite Knochenschraubenelement (2) im ersten Längsschlitz (18) aufgenommen ist.

7. Knochenschraube nach einem der vorhergehenden Ansprüche, wobei das erste Knochenschraubenelement (1) und das zweite Knochenschraubenelement (2) als ein spiralförmiges Plattenelement ausgebildet sind.

8. Knochenschraube nach einem der vorhergehenden Ansprüche, wobei das erste und zweite Knochenschraubenelement (1, 2) kreuzweise zueinander angeordnet sind.

9. Knochenschraube nach einem der vorhergehenden Ansprüche, wobei ein drittes Knochenschraubenelement (3) vorgesehen ist, wobei das erste, zweite und dritte Knochenschraubenelement (1, 2, 3) sternförmig zueinander angeordnet sind.

10. Knochenschraube nach einem der vorhergehenden Ansprüche, wobei das Gewindeprofil eine vordere Gewindeflanke und eine hintere Gewindeflanke enthält, wobei die vordere Gewindeflanke einen spitzeren Winkel als die hintere Gewindeflanke aufweist.

11. Knochenschraube nach einem der vorhergehenden Ansprüche, wobei das Gewindeprofil als Sägezahnprofil ausgebildet ist.

12. Knochenschraube nach einem der vorhergehenden Ansprüche, welche ein biokompatibles Material enthält.

13. Knochenschraube nach einem der vorhergehenden Ansprüche, wobei die Knochenschraubenelemente formschlüssig miteinander verbunden sind.

14. Knochenschraube nach einem der vorhergehenden Ansprüche, wobei die Knochenschraubenelemente mittels eines Fügeverfahrens materialschlüssig verbunden sind.

15. Knochenschraube nach einem der vorhergehenden Ansprüche, wobei das Schraubenkopfelement (15, 25, 35) eine maximale Breite aufweist, die grösser ist als die maximale Breite des Gewindeprofils.

## Claims

1. A bone screw (10, 20, 30) containing a first bone screw element (1), a second bone screw element (2, 3), wherein the first bone screw element (1) and the second bone screw element (2, 3) are formed as a thin-walled plate element wherein the thin-walled plate element has a wall thickness that is less than 10% of the length of the plate element, wherein each of the first or second bone screw elements (1, 2, 3) comprises a screw head element (15, 25, 35) and a screw shaft element (14, 24 , 34), **characterized in that** the screw shaft element (14, 24, 34) has a first side flank (16, 26, 36) and a second side flank (17, 27, 37), whereby each of the side flanks have a threaded profile.

2. The bone screw according to claim 1, wherein the end of the bone screw element (1, 2, 3) opposite to the screw head element (15, 25, 35) is configured as a screw tip element (12, 22, 32).

3. The bone screw according to one of claims 1 or 2, wherein the first bone screw element and the second bone screw element (1, 2, 3) have a first and a second longitudinal slot (18, 28, 38, 48) which do not extend over the entire length of the bone screw element.

4. The bone screw of claim 3, wherein the longitudinal slot (18) of the first bone screw element (1) extends from the screw tip member (12) to a point of intersection (19).

5. The bone screw of one of claims 3 or 4, wherein the longitudinal slot of the second bone screw element (2) extends from the screw head element (25) to a point of intersection (29).

6. The bone screw of claim 5, wherein the first bone screw element (1) is received in the second longitudinal slot (28) and the second bone screw element (2) is received in the first longitudinal slot (18).

7. The bone screw according to one of the preceding claims, wherein the first bone screw element (1) and the second bone screw element (2) is formed as a spiral plate element.

8. The bone screw according to one of the preceding claims, wherein the first and second bone screw element (1, 2) are arranged crosswise with respect to each other.

9. The bone screw according to one of the preceding claims, wherein a third bone screw element (3) is provided, wherein the first, second and third bone screw element (1, 2, 3) are arranged star-shaped with respect to each other.

10. The bone screw according to one of the preceding claims, wherein the threaded profile comprises a front threaded flank and a rear threaded flank, wherein the front threaded flank has a more acute angle than the rear threaded flank.

11. The bone screw according to one of the preceding claims, wherein the threaded profile comprises a saw tooth profile.

12. The bone screw according to one of the preceding claims, comprising a biocompatible material.

13. The bone screw according to one of the preceding claims, wherein the bone screw elements are positively connected with each other.

14. The bone screw according to one of the preceding claims, wherein the bone screw elements are materially connected by means of a joining method.

15. The bone screw according to one of the preceding claims, wherein the screw head element (15, 25, 35) has a maximal width which is greater than the maximal width of the threaded profile.

## Revendications

1. Vis à os (10, 20, 30) contenant un premier élément de vis à os (1), un deuxième élément de vis à os (2, 3), le premier élément de vis à os (1) et le deuxième élément de vis à os (2, 3) sont formés en tant qu'élément de plaque à paroi mince, en ce que l'élément de plaque à paroi mince a une épaisseur de paroi inférieure de 10% de la longueur de l'élément de plaque, en ce que chacun des premier ou deuxième éléments de vis à os (1, 2, 3) comprend un élément de tête de vis (15, 25, 35) et un élément de tige de vis (14, 24, 34), **caractérisé en ce que** l'élément de tige de vis (14, 24, 34) présente un premier flanc latéral (16, 26, 36) et un deuxième flanc latéral (17, 27, 37), chacun des flancs latéraux ayant un profil fileté.

2. Vis à os selon la revendication 1, dans laquelle ledit élément de tête de vis (15, 25, 35) opposé à l'extrémité de l'élément de vis à os (1, 2, 3) est formé en tant qu'élément de pointe de vis (12, 22, 32).

3. Vis à os selon une des revendications 1 ou 2, dans laquelle le premier élément de vis à os et le deuxième élément de vis à os (1, 2, 3) ont une première et une deuxième fente longitudinale (18, 28, 38, 48) qui ne s'étendent pas sur toute la longueur de l'élément de la vis à os.

4. Vis à os selon la revendication 3, dans laquelle la fente longitudinale (18) du premier élément de vis à os (1) s'étend de l'élément de pointe de vis (12) jusqu'à un point d'intersection (19).

5. Vis à os selon l'une des revendications 3 ou 4, dans laquelle la fente longitudinale du deuxième élément de vis à os (2) s'étend de l'élément de tête de vis (25) jusqu'à un point d'intersection (29).

6. Vis à os selon la revendication 5, dans laquelle le premier élément de vis à os (1) est reçu dans la deuxième fente longitudinale (28) et le deuxième élément de vis à os (2) est reçu dans la première fente longitudinale (18).

7. Vis à os selon l'une des revendications précédentes, dans laquelle le premier élément de vis à os (1) et le deuxième élément de vis à os (2) sont formés en tant qu'élément de plaque en forme de spirale.

8. Vis à os selon l'une des revendications précédentes, dans laquelle les premier et deuxièmes éléments de vis à os (1, 2) sont disposés en croix l'un par rapport à l'autre.

9. Vis à os selon l'une des revendications précédentes, dans laquelle est prévu un troisième élément de vis à os (3), dans laquelle les premiers, deuxièmes et troisièmes éléments de vis à os (1, 2, 3) sont disposés en forme d'étoile l'un par rapport à l'autre.

10. Vis à os selon l'une des revendications précédentes, dans laquelle le profil fileté comprend un flanc fileté avant et un flanc fileté arrière, dans lequel le flanc fileté avant a un angle plus aigu que le flanc fileté arrière.

11. Vis à os selon l'une des revendications précédentes, dans laquelle le profil fileté comprend un profil en dents de scie.

12. Vis à os selon l'une des revendications précédentes, comprenant un matériau biocompatible.

13. Vis à os selon l'une des revendications précédentes, dans laquelle les éléments de vis à os sont reliés positivement les uns aux autres.

14. Vis à os selon l'une des revendications précédentes, dans laquelle les éléments de vis à os sont reliés matériellement par un procédé de jonction.

15. Vis à os selon l'une des revendications précédentes, dans laquelle l'élément de tête de vis (15, 25, 35) a une largeur maximale qui est supérieure à la largeur maximale du profil fileté.
